# EUROPÄISCHE PATENTSCHRIFT

(11) **EP 0 673 918 B1**
(45) Veröffentlichungstag und Bekanntmachung des Hinweises auf die Patenterteilung: **02.06.1999**
(21) Anmeldenummer: 95102940.4
(22) Anmeldetag: 02.03.1995
(51) Int. Cl.: C07C 209/48

(54) **Verfahren zur Herstellung von peralkylierten Aminen**
Process for the preparation of peralkylated amines
Procédé de préparation d'amines peralkylées

(30) Priorität: 07.03.1994 DE 4407466
(43) Veröffentlichungstag der Anmeldung: 27.09.1995
(73) Patentinhaber: BASF AKTIENGESELLSCHAFT, 67056 Ludwigshafen (DE)
(72) Erfinder: Witzel, Tom, Dr., D-67069 Ludwigshafen (DE); Fuchs, Eberhard, Dr., D-67227 Frankenthal (DE); Zimmermann, Horst, Dr., D-68199 Mannheim (DE)
(74) Vertreter: Kinzebach, Werner, Dr.

(56) Entgegenhaltungen:
- EP-A- 0 599 180
- DE-A- 3 935 641
- STUDIES IN SURFACE SCIENCE AND CATALYSIS, Bd. 27, 1986 Seiten 105-144, VOLF, J. & PASEK, J. 'Hydrogenation of Nitriles'

## Beschreibung

Die vorliegende Erfindung betrifft ein Verfahren zur Herstellung peralkylierten Aminen aus Nitrilen und sekundären Aminen an einem Palladium-Katalysator bei erhöhten Temperaturen und Drücken.

In Stud. Surf. Sci. Catal., 27 (1986) 105 bis 144 ist auf Seite 123 die Bildung tertiärer Amine aus sekundären Aminen und einem aliphatischen Nitril an einem Pd/C-Katalysator beschrieben, jedoch wird das Verfahren mit dem Vorurteil verworfen, daß die Reaktionsgeschwindigkeit mit steigender Startkonzentration des sekundären Amins sinkt und nur eine unbefriedigende Ausbeute erreicht werden.

Auch nach Ind. Tech. Bull., 11 (1970) 19 bis 24 läßt sich eine Synthese tertiärer Amine aus einem sekundären Amin und einem Nitril in technisch relevanter Ausbeute nicht erwarten. Zwar bildet sich das tertiäre Amin Tripentylamin aus Valeronitril an Pd/C mit 84 % Selektivität, der Umsatz beläuft sich aber nur auf unbefriedigende 28 %. Dagegen erhält man am gleichen Katalysator aus Valeronitril und Butylamin das sekundäre Butylpentylamin in 93 % bei 54 % Umsatz. Trotz vorhandenem sekundären Amin wird aber kein tertiäres Amin gebildet.

In Catalysis of Organic Reactions, Marcel Dekker, New York, Basel, 1992, Seite 103 wird die Verwendung von Katalysatorträgern wie Aluminiumoxid zur Herstellung von primären Aminen empfohlen, da die sauren Zentren des Trägers bereits gebildetes Amin abseits der aktiven Zentren adsorbieren und somit eine Verknüpfung verhindern.

Aus GB-A-1 157 637, GB-A-1 157 638 und GB-A-1 157 639 ist die Reaktion von 2-Methylglutarsäuredinitril mit Diethylamin in Gegenwart von Wasserstoff und Palladium auf Bariumsulfat oder bevorzugt Palladium auf Kohle zum 5-Diethylamino-2-methylvalero-nitril bekannt. Trotz langer Reaktionszeit und einem Diethylaminüberschuß von 200 mol% wurde kein Tetraethylderivat gefunden, obwohl die Diethylaminogruppe nicht nur mit der Nitrilgruppen in 5-Position, sondern auch mit der in 1-Position reagiert (Verhältnis 4 zu 1).

Aus der DE-A-39 35 641 ist die Synthese eines sekundären Amins an einem Palladium-Katalysator beschrieben. Hier erfolgt die Reaktion von Dimethylaminopropionitril mit sich selbst unter Hydrierbedingungen zum Bis-(3-Dimethylaminopropyl)-amin. Die Bildung eines tertiären Amins wird lediglich an einem wesentlich aufwendiger herzustellenden Spinell als Trägermaterial in max. 58 % Ausbeute erreicht.

Aus der US-A-2 166 183 wird bei der Hydrierung von Dinitrilen mit 4, 5 oder 6 Kohlenstoffatomen vor der Bildung cyclischer sekundärer Amine gewarnt; im Falle von Adipodinitril also vor Hexamethylen-imin.

In Chem.Prum., 56, (1981) 349-356 wird die Nitrilhydrierung mit Palladiumkatalysatoren, insbesondere der Einfluß des Trägers auf die Eigenschaften der Palladiumkatalysatoren, untersucht. Anhand der Hydrierung von Acetonitril wird festgestellt, daß der Träger-Typ die Selektivität der Palladiumkatalysatoren nicht beeinflusst; es ist allerdings ersichtlich, daß die Reaktionsgeschwindigkeit bei der Verwendung von Pd/C deutlich höher ist als bei Verwendung von Pd/Al₂O₃.

Der vorliegenden Erfindung lag die Aufgabe zugrunde, ein verbessertes Verfahren zur Herstellung peralkylierter Amine zur Verfügung zu stellen.

Gelöst wurde diese Aufgabe durch ein Verfahren zur Herstellung von peralkylierten Aminen der allgemeinen Formel I 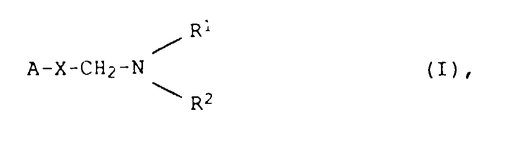 in der
- R¹, R²: C₁- bis C₂₀₀-Alkyl, C₃- bis C₈-Cycloalkyl, C₄- bis C₂₀-Alkylcycloalkyl, C₄- bis C₂₀-Cycloalkyl-alkyl, C₂- bis C₂₀-Alkoxyalkyl, Aryl, C₇- bis C₂₀-Alkylaryl, C₇- bis C₂₀-Aralkyl, C₂- bis C₈-Hydroxyalkyl, C₂- bis C₈-Mercaptoalkyl, C₈- bis C₂₀-Phenoxyalkyl, C₂- bis C₈-Aminoalkyl, C₂- bis C₈-(NHR⁴)-alkyl, C₂- bis C₈-(NR⁴R⁵)-alkyl oder gemeinsam eine gegebenenfalls durch ein- bis dreifach durch C₁- bis C₄-Alkyl substituierte gesättigte oder ungesättigte gegebenenfalls durch Sauerstoff oder Stickstoff unterbrochene C₂- bis C₆-Alkylenkette
- X: eine gegebenenfalls ein- bis fünffach durch R³, C₁- bis C₈-Alkyl, C₁- bis C₈-Alkoxy, C₁- bis C₈-Dialkylamino, Phenoxy, Diphenylamino und/oder C₂- bis C₈-Alkoxycarbonyl substituiertes C₂- bis C₂₀-Alkylen oder C₂- bis C₂₀-Alkenylen oder C₄- bis C₈-Cycloalkylen,
- A: Wasserstoff, C₁- bis C₂₀-Alkyl, C₃- bis C₈-Cycloalkyl, C₄- bis C₂₀-Alkyl-cycloalkyl, C₄- bis C₂₀-Cycloalkyl-alkyl, C₂- bis C₂₀-Alkoxyalkyl, Aryl, C₇- bis C₂₀-Alkylaryl, C₇- bis C₂₀-Aralkyl, C₁- bis C₂₀-Alkoxy, Hydroxy, C₁- bis C₂₀-Hydroxyalkyl, Amino, C₁- bis C₂₀-Alkylamino, C₂- bis C₂₀-Dialkylamino, C₃- bis C₁₂-Alkenylenamino, C₃- bis C₈-Cycloalkylamino, Arylamino, Aryl-C₁- bis C₈-alkylamino, Halogen, Mercapto, C₂- bis C₂₀-Alkenylenoxy, C₃- bis C₈-Cycloalkoxy und Aryloxy
- R³: CH₂-NR¹R²,
- R⁴, R⁵: C₁- bis C₂₀-Alkyl bedeuten,
wobei man ein Nitril der allgemeinen Formel II

A - X - CN (II),

in denen R¹, R², R⁴, R⁵, X und A die oben genannten Bedeutungen haben und R³ für -CH₂-NR¹R² oder Cyano steht, zu einem sekundären Amin der allgemeinen Formel III 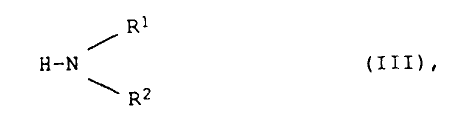 gibt, und das erhaltende Gemisch mit Wasserstoff bei Temperaturen von 50 bis 250°C und Drücken von 5 bis 350 bar in Gegenwart von Palladium als Katalysator umsetzt, das dadurch gekennzeichnet ist, daß man das Palladium auf oxidischen Trägern einsetzt, die ausgewählt sind unter γ-Al₂O₃, α-Al₂O₃, SiO₂, TiO₂ oder ZrO₂ oder mit Alkali oder Erdalkali dotiertes γ-Al₂O₃, α-Al₂O₃, SiO₂, TiO₂ oder ZrO₂.

Das erfindungsgemäße Verfahren läßt sich wie folgt durchführen:

Die Umsetzung der Nitrile II mit den sekundären Aminen III mit Wasserstoff kann bei Temperaturen von 50 bis 250°C, bevorzugt 90 bis 200°C, besonders bevorzugt 120 bis 160°C und Drücken von 5 bis 350 bar, bevorzugt 50 bis 200 bar, besonders bevorzugt 70 bis 150 bar diskontinuierlich oder bevorzugt kontinuierlich in Druckapparaturen wie Autoklaven oder Rohrreaktoren oder deren Kombinationen oder bevorzugt in einem Rohrreaktor an bestimmten Hydrierkatalysatoren durchgeführt werden.

Als Hydrierkatalysatoren eignen sich Palladium-Katalysatoren auf oxidischen Trägern. Als oxidische Träger eignen sich beispielsweise gamma-Al₂O₃, α-Al₂O₃, SiO₂, TiO₂, ZrO₂, mit Alkali- oder Erdalkalioxiden dotiertes α- oder γ-Al₂O₃, SiO₂, TiO₂ oder ZrO₂.

Diese Palladium-Katalysatoren enthalten in der Regel 0,1 bis 10 Gew.-%, bevorzugt 0,3 bis 5 Gew.-% Palladium, besonders bevorzugt 0,5 bis 1 Gew.-%, bezogen auf das Gesamtgewicht des Katalysators.

Die Palladium-Katalysatoren sind allgemein bekannt oder können nach allgemein bekannten Verfahren, beispielsweise durch Tränken des Trägers mit Palladiumverbindungen wie PdCl₂ oder Pd(NO₃)₂, hergestellt werden.

Das Molverhältnis von sekundäre Amin III zum Nitril II beträgt in der Regel 1 : 1 bis 30 : 1, bevorzugt 1 : 1 bis 15 : 1, besonders bevorzugt 1,1 : 1 bis 5 : 1. Es können jedoch auch größere Aminüberschüsse oder auch Aminunterschüsse eingestellt werden.

Das erfindungsgemäße Verfahren läßt sich lösungsmittelfrei oder in Lösungsmitteln wie Wasser, Methanol, Ethanol, Tetrahydrofuran, Methyl-tert.-butylether durchführen. Im Lösungsmittel kann dabei auch Ammoniak oder das sekundäre Amin IIIgelöst sein.

Die im erfindungsgemäßen Verfahren entstehenden peralkylierten Amine I lassen sich in an sich bekannter Weise, beispielsweise destillativ, reinigen.

Das Zwischenglied X und die Substituenten A, R¹, R², R⁴ und R⁵ in den Verbindungen I, II und III haben unabhängig voneinander folgende Bedeutungen:
R¹, R²
   - C₁- bis C₂₀₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
A, R⁴, R⁵
   - C₁- bis C₂₀-Alkyl, bevorzugt C₁- bis C₁₂-Alkyl wie Methyl, Ethyl, n-Propyl, iso-Propyl, n-Butyl, iso- Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
R¹, R², A
   - C₃- bis C₈-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
   - C₄- bis C₂₀-Alkyl-cycloalkyl, bevorzugt C₄- bis C₁₂-Alkylcycloalkyl,
   - C₄- bis C₂₀-Cycloalkyl-alkyl, -alkyl, bevorzugt C₄- bis C₁₂-Cycloalkylalkyl,
   - C₂- bis C₂₀-Alkoxyalkyl, bevorzugt C₂- bis C₈-Alkoxyalkyl wie Methoxymethyl, 2-Methoxy-ethyl, 2-Ethoxyethyl, 3-Methoxypropyl und 3-Ethoxypropyl,
   - Aryl wie Phenyl, 1-Naphthyl und 2-Naphthyl, bevorzugt Phenyl,
   - C₇- bis C₂₀-Alkylaryl wie C₇- bis C₂₀-Phenylalkyl, bevorzugt C₇- bis C₁₂-Phenylalkyl wie Benzyl, 1-Phenethyl, 2-Phenethyl, 1-Phenyl-propyl, 2-Phenylpropyl, 3-Phenyl-propyl, 1-Phenylbutyl, 2-Phenyl-butyl, 3-Phenyl-butyl und 4-Phenyl-butyl, besonders bevorzugt Benzyl, 1-Phenethyl und 2-Phenethyl,
   - C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R¹, R²
   - C₂- bis C₈-Hydroxyalkyl, bevorzugt C₂- bis C₄-Hydroxyalkyl wie 1-Hydroxyethyl, 2-Hydroxyethyl, 2-Hydroxy-n-propyl und 3-Hydroxy-n-propyl,
   - C₂- bis C₈-Mercaptoalkyl, bevorzugt C₂- bis C₄-Mercaptoalkyl wie 1-Mercaptoethyl, 2-Mercaptoethyl, 2-Mercapto-n-propyl und 3-Mercapto-n-propyl,
   - C₈- bis C₂₀-Phenoxyalkyl, bevorzugt C₈- bis C₁₂-Phenoxyalkyl wie 2-Phenoxyethyl, 2-Phenoxy-propyl, 3-Phenoxy-propyl, 2-Phenoxybutyl, 3-Phenoxy-butyl und 4-Phenoxy-butyl, besonders bevorzugt 2-Phenoxyethyl,
   - C₂- bis C₈-Aminoalkyl, bevorzugt C₂- bis C₄-Aminoalkyl wie 1-Aminoethyl, 2-Aminoethyl, 2-Amino-n-propyl und 3-Amino-n-propyl,
   - C₂- bis C₈-(NHR⁴)-alkyl, bevorzugt C₂- bis C₄-(NHR⁴)-alkyl wie (NHR⁴)-methyl, (NHR⁴)-1-ethyl und (NHR⁴)-2-ethyl,
   - C₂- bis C₈-(NR⁴R⁵)-alkyl, bevorzugt C₂- bis C₄-(NR⁴R⁵)-alkyl wie (NR⁴R⁵)-methyl, (NR⁴R⁵)-1-ethyl und (NR⁴R⁵)-2-ethyl oder
   - gemeinsam eine gegebenenfalls durch ein- bis dreifach durch C₁- bis C₄-Alkyl substituierte gesättigte oder ungesättigte gegebenenfalls durch Sauerstoff oder Stickstoff unterbrochene C₂- bis C₆-Alkylenkette wie -CH₂-O-CH₂-CH₂-, -CH₂-CH₂-O-CH₂-CH₂-, -CH₂-CH₂-O-CH₂-CH₂-CH₂-, -CH₂-CH₂-CH₂-O-CH₂-CH₂-CH₂-, -CH₂-N-CH₂-CH₂-,-CH₂-CH₂-N-CH₂-CH₂-, -CH₂-CH₂-N-CH₂-CH₂-CH₂-, -CH₂-CH₂-CH₂-N-CH₂-CH₂-CH₂-, -CH₂-CH₂-NH-CH₂-CH₂-CH₂-CH₂-N(CH₃)-CH₂-CH₂-, -CH₂-CH₂-N(CH₂CH₂)-CH₂-CH₂- und -CH₂-CH(CH₃)-O-CH(CH₃)-CH₂,
X
   - eine gegebenenfalls ein- bis fünffach durch R³, C₁- bis C₈-Alkyl, C₁- bis C₈-Alkoxy, C₁- bis C₈-Dialkylamino, Phenoxy, Diphenylamino und/oder C₂- bis C₈-Alkoxycarbonyl substituiertes C₂- bis C₂₀-Alkylen oder C₂- bis C₂₀-Alkenylen oder C₄- bis C₈-Cycloalkylen, bevorzugt eine gegebenenfalls ein- bis dreifach durch C₁- bis C₈-Alkyl, substituiertes C₂- bis C₈-Alkylen wie -(CH₂)₂-, -(CH₂)₃-, -(CH₂)₄-, -(CH₂)₅-, -(CH₂)₆-, -( CH₂)₇-, -(CH₂)₈-, - CH(CH₃)-CH₂-CH₂-, -CH₂-CH(CH₃)-CH₂-, -CH₂-C(CH₃)₂-CH₂-, bevorzugt -(CH₂)₂-, -(CH₂)₃-, -(CH₂)₄-, -(CH₂)₆-, -CH₂-CH(CH₃)-CH₂-CH₂-, -CH₂-C(CH₂)-CH₂-CH₂, -CH₂-CH₂-CH(CN)-CH₂-CH₂-CH₂, besonders bevorzugt -(CH₂)₄-,
A
   - Wasserstoff,
   - C₁- bis C₂₀-Alkoxy, bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy,
   - Hydroxy,
   - C₁- bis C₂₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 2-Hydroxy-n-propyl und 3-Hydroxy-n-propyl,
   - Amino,
   - C₁- bis C₂₀-Alkylamino, bevorzugt C₁- bis C₈-Aminoalkyl, besonders bevorzugt C₁- bis C₄-Aminoalkyl wie Methylamino, 1-Aminoethyl, 2-Aminoethyl, 2-Amino-n-propyl und 3-Amino-n-propyl,
   - C₂- bis C₂₀-Dialkylamino, bevorzugt C₂- bis C₁₂-alkylamino, besonders C₂- bis C₈-Dialkylamino wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methyl-propyl)amino, N-Methyl-N-(2-methylpropyl)amino, N-(1,1-Dimethyl-ethyl)-N-methylamino, N-Ethyl-N-propylamino, N-Ethyl-N-(1-methyl-ethyl)-amino, N-Butyl-N-ethylamino, N-Ethyl-N-(1-methylpropyl)amino, N-Ethyl-N-(2-methylpropyl)amino, N-Ethyl-N-(1,1-dimethylethyl)-amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino,
   - C₃- bis C₁₂-Azacycloalkyl, bevorzugt C₃- bis C₈-Azacycloalkylamino, besonders bevorzugt C₅- bis C₈-Azacycloalkyl wie Pyrrolidin, Piperidin, Azepan, Piperazin, N-Alkylpiperazin und Morpholin,
   - C₃- bis C₈-Cycloalkylamino wie Cyclopropylamino, Cyclobutylamino, Cyclopentylamino, Cyclohexylamino, Cycloheptylamino und Cyclooctylamino, bevorzugt Cyclopentylamino, Cyclohexylamino und Cyclooctylamino, besonders bevorzugt Cyclopentylamino und Cyclohexylamino,
   - C₃- bis C₈-Dicycloalkylamino,
   - Arylamino wie Phenylamino, 1-Naphthylamino und 2-Naphthylamino, bevorzugt Phenylamino,
   - Aryl-C₁- bis C₈-alkylamino, bevorzugt Phenyl-C₁- bis C₈-alkylamino, besonders bevorzugt Phenyl-C₁- bis C₄-alkylamino wie Phenyl/Methyl-amino und Phenyl/Ethylamino,
   - Halogen, bevorzugt Fluor, Chlor und Brom, besonders bevorzugt Fluor und Chlor,
   - Mercapto, -SH,
   - C₂- bis C₂₀-Oxacycloalkyl, bevorzugt C₂- bis C₈-Oxacycloalkyl, besonders bevorzugt C₂- bis C₈-Oxacycloalkyl, wie 2-Tetrahydrofuranyl, 3-Tetrahydrofuranyl, 2-Furanyl und 3-Furanyl,
   - C₃- bis C₈-Cycloalkoxy wie Cyclopropoxy, Cyclobutoxy, Cyclopentoxy, Cyclohexoxy, Cycloheptoxy und Cyclooctoxy, bevorzugt Cyclopentoxy, Cyclohexoxy und Cyclooctoxy, besonders bevorzugt Cyclopentoxy und Cyclohexoxy,
   - Aryloxy wie Phenoxy, 1-Naphthoxy und 2-Naphthoxy, bevorzugt Phenoxy.

Der Substituent R³ bedeutet in den Verbindungen I -CH₂-NR¹R², in den Verbindungen II steht der Substituent R³ für -CH₂-NR¹R² oder Cyano, bevorzugt Cyano.

Bevorzugt sind solche Verbindungen I und II, in denen A ungleich Wasserstoff ist, wenn X eine -C(CN)-Gruppe in α-Stellung zum Substituenten A trägt.

Bevorzugte Nitrilverbindungen II sind:
Acetonitril, Propionitril, Isopropionitril, Valeronitril, Pentensäurenitril, Retensäurenitril, 3-Hydroxypropionitril, 3-Methoxypropionitril, 3-Ethoxypropionitril, 3-Propoxypropionitril, 3-Isopropoxypropionitril, 3-Cyclohexoxypropionitril, 2-Methyl-3-hydroxypropionitril, 3- Methoxy-2-methyl-propionitril, 3- Ethoxy-2-methyl-propionitril, 2-Methyl-3-propoxypropionitril, 3-Isopropoxy-2-methyl-propionitril, 3-Cyclohexoxy-2-methyl-propionitril, 3-Methyl-3-hydroxypropionitril, 3- Methoxy-3-methyl-propionitril, 3- Ethoxy-3-methyl-propionitril, 3-Methyl-3-propoxypropionitril, 3-Isopropoxy-3-methyl-propionitril, 3-Cyclohexoxy-3-methyl-propionitril, 3-Aminopropionitril, 3-Methylaminopropionitril, 3-Dimethyl-aminopropionitril, 3-Ethylaminopropionitril, 3-Diethylaminopropio-nitril, 3-Propylaminopropionitril, 3-Dipropylaminopropionitril, 3-Isopropylaminopropionitril, 3-Diisopropylaminopropionitril, 3-Cyclohexylaminopropionitril, 3-Dicyclohxylaminopropionitril, N-(Cyanoethyl)-N-methylanilin. Besonders bevorzugt sind 3-Hydroxy-propionitril, 3- Methoxypropionitril, 3-Dimethylaminopropionitril, 3-Diethylaminopropionitril, 3-Cyclohexylaminopropionitril und 3-Methylaminopropionitril, bevorzugt Biscyanethylether, Biscyanethylamin, N-Methyl-biscyanethylamin, N-Ethylbiscyanethylamin, N-n-Propyl-biscyanethylamin, N-n-Propyl-biscyanethylamin, Polyisobutylennitril, N-Polyisobutylenaminopropionitril, Triscyanethylamin, 5-Aminovaleriansäurenitril, 5-Methylaminovaleriansäurenitril, 5-Dimethylaminovaleriansäurenitril, 6-Aminocapronsäurenitril, 6-Methylaminocapronsäurenitril, 6-Dimethylaminocapronsäurenitril, 5-Amino-4-methylvaleriansäurenitril, 5-Methylamino-4-methylvaleriansäurenitril, 5-Dimethylamino-4-methylvaleriansäurenitril, 5-Ethylamino-4-methylvaleriansäurenitril, 5-Diethylamino-4-methylvaleriansäurenitril, 5-Amino-2-methylvaleriansäurenitril, 5-Methylamino-2-methylvaleriansäurenitril, 5-Dimethylamino-2-valeriansäurenitril, 5-Ethylamino-2-methylvaleriansäurenitril, 5-Diethylamino-2-methylvaleriansäurenitril, 4-Cyanokorksäuredinitril.

Bevorzugte sekundäre Amine III sind:
Dimethylamin, Diethylamin, Dipropylamin, Diisopropylamin, Di-n-butylamin, Diisobutylamin, Di-sec.-butylamin, Di-2-ethylhexylamin, Di-tridecylamin, Dicyclohxylamin, Ethyl-methylamin, Methylcyclo-hexylamin, Ethyl-cyclohexylamin, Piperazin, N-Methylpiperzin, N-Ethylpiperazin, Diphenylamin, N-Methylanilin, N-Ethylanilin, Di-ethanolamin, Di-2-methoxyethylamin, Di-2-ethoxyethylamin, Methyl-ethanolamin, Ethylethanolamin, Isopropylethanolamin, Hydroxy-ethylanilin. Besonders bevorzugt sind Dimethylamin, Diethylamin und Piperazin.

Die tertiären Amine I sind Härter für Epoxyharze, Katalysatoren für Polyurethane, Zwischenprodukte zur Herstellung quartärer Ammoniumverbindungen, Weichmacher, Korrosionsinhibitoren, Textilhilfs-mittel, Farbstoffe und Emulgatoren. Mehrfach funktionalisierte tertiäre Amine dienen außerdem zur Herstellung von Kunstharzen, Ionenaustauschern, Pharmazeutika, Pflanzenschutz- und Schädlings-bekämpfungsmitteln.

### Beispiele

- Katalysator 1:: 0,5 Gew.-% Pd auf Al₂O₃ mit 20 Gew.-% CaO
- Katalysator 2:: 0,5 Gew.-% Pd; 5 Gew.-% Pr auf Al₂O₃
- Katalysator 3:: 0,5 Gew.-% Pd auf Al₂O₃

### Beispiel 1

Durch einen vertikalen, mit 39 g des Katalysators 2 gefüllten, Hydrierreaktor (Durchmesser: 16 mm; Füllhöhe: 600 mm; ölbeheizter Doppelmantel), wurde in Sumpffahrweise bei 80 bar und 150°C stündlich 10,1 ml 3-Dimethylaminopropionitril und 10 ml flüssiges Dimethylamin (Molverhältnis 1 : 2,5) gepumpt. Gleichzeitig leitete man 10 Nl/h Wasserstoff von unten nach oben durch den Reaktor. Nach Entspannen auf Normaldruck und Entfernen des überschüssigen Dimethylamins erhielt man nach Destillation stündlich 10,2 g (88 %) Tetramethylpropylendiamin, Sdp.: 144 bis 148°C.

### Beispiel 2

In der in Beispiel 1 beschriebenen Apparatur erhielt man an 47 g Katalysator 2 bei 80 bar und 150°C aus stündlich 18,4 ml 3-Hydroxypropionitril und 23 ml Dimethylamin (Molverhältnis 1 : 2,5) stündlich 22 g (83 %) 3-Dimethylaminopropanol, Sdp.: 82 bis 84°C/5 mbar.

### Beispiel 3

In der in Beispiel 1 beschriebenen Apparatur erhielt man an 39 g Katalysator 1 bei 80 bar und 155°C aus stündlich 7,6 ml 3-Hydroxypropionitril und 20 ml Dimethylamin (Molverhältnis 1 : 2,5) stündlich 9,7 g (89 %) 3-Dimethylaminopropanol, Sdp.: 82 bis 84°C/5 mbar.

### Beispiel 4

In einer Technikumsapparatur entsprechend der in Beispiel 1 beschriebenen Apparatur (Katalysatorvolumen 800 ml) erhielt man an 650 g Katalysator 3 bei 200 bar und 140°C aus stündlich 160 ml 3-Hydroxypropionitril und 193 ml Dimethylamin (Molverhältnis 1 : 1,2) stündlich 24,5 g (93 %) 3-Dimethylaminopropanol, Sdp.: 82 bis 84°C/5 mbar.

### Beispiel 5

In einem 300 ml Autoklaven wurden 43 g (0,5 mol) Piperazin und 14 g 25 %ige Ammoniumhydroxid-Lösung und 31 g (0,75 mol) Acetonitril an 10 g des Katalysators 2 bei 100°C unter 80 bar Wasserstoff hydriert. Die Monoselektivität erreichte bei 53 % Piperazinumsatz 96 %. Wurde die Reaktion bis zu einem Umsatz von 100 % gefahren, so betrug die Selektivität 65 %. Der Katalysator konnte mehrmals ohne Ausbeuteverluste eingesetzt werden.

## Patentansprüche

1. Verfahren zur Herstellung von peralkylierten Aminen der allgemeinen Formel I 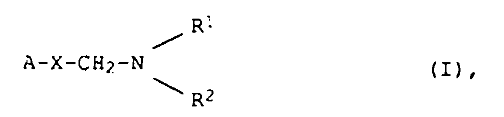
R¹, R² C₁- bis C₂₀₀-Alkyl, C₃- bis C₈-Cycloalkyl, C₄- bis C₂₀-Alkyl-cycloalkyl, C₄- bis C₂₀-Cycloalkyl-alkyl, C₂- bis C₂₀-Alkoxyalkyl, Aryl, C₇- bis C₂₀-Alkylaryl, C₇- bis C₂₀-Aralkyl, C₂- bis C₈-Hydroxyalkyl, C₂- bis C₈-Mercaptoalkyl, C₈- bis C₂₀-Phenoxyalkyl, C₂- bis C₈-Aminoalkyl, C₂- bis C₈-(NHR⁴)-alkyl, C₂- bis C₈-(NR⁴R⁵)-alkyl oder gemeinsam eine gegebenenfalls durch ein- bis dreifach durch C₁- bis C₄-Alkyl substituierte gesättigte oder ungesättigte gegebenenfalls durch Sauerstoff oder Stickstoff unterbrochene C₂- bis C₆-Alkylenkette
X eine gegebenenfalls ein- bis fünffach durch R³, C₁- bis C₈-Alkyl, C₁- bis C₈-Alkoxy, C₁- bis C₈-Dialkylamino, Phenoxy, Diphenylamino und/oder C₂- bis C₈-Alkoxycarbonyl substituiertes C₂- bis C₂₀-Alkylen oder C₂- bis C₂₀-Alkenylen oder C₄- bis C₈-Cycloalkylen,
A Wasserstoff, C₁- bis C₂₀-Alkyl, C₃- bis C₈-Cycloalkyl, C₄- bis C₂₀-Alkyl-cycloalkyl, C₄- bis C₂₀-Cycloalkyl-alkyl, C₂- bis C₂₀-Alkoxyalkyl, Aryl, C₇- bis C₂₀-Alkylaryl, C₇- bis C₂₀-Aralkyl, C₁- bis C₂₀-Alkoxy, Hydroxy, C₁- bis C₂₀-Hydroxyalkyl, Amino, C₁- bis C₂₀-Alkylamino, C₂- bis C₂₀-Dialkylamino, C₃-bis C₁₂-Alkenylenamino, C₃- bis C₈-Cycloalkylamino, Arylamino, Aryl-C₁- bis C₈-alkylamino, Halogen, Mercapto, C₂- bis C₂₀-Alkenylenoxy, C₃- bis C₈-Cycloalkoxy und Aryloxy
R³ CH₂-NR¹R²,
R⁴, R⁵ C₁- bis C₂₀-Alkyl bedeuten,
wobei man ein Nitril der allgemeinen Formel II
A - X - CN (II),
worin X und A die oben genannten Bedeutungen haben zu einem sekundären Amin der allgemeinen Formel III 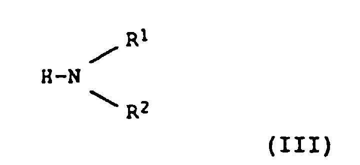 worin R¹ und R² die oben genannten Bedeutungen haben, wobei R³ für -CH₂-NR¹R² oder Cyano steht, gibt, und das erhaltene Gemisch mit Wasserstoff bei Temperaturen von 50 bis 250°C und Drücken von 5 bis 350 bar in Gegenwart von Palladium als Katalysator umsetzt, dadurch gekennzeichnet, daß man das Palladium auf oxidischen Trägern einsetzt, die ausgewählt sind unter γ-Al₂O₃, α-Al₂O₃, SiO₂, TiO₂ oder ZrO₂ oder mit Alkali oder Erdalkali dotiertes γ-Al₂O₃, α-Al₂O₃, SiO₂, TiO₂ oder ZrO₂.

2. Verfahren nach Anspruch 1, dadurch gekennzeichnet, daß A ungleich Wasserstoff ist, wenn X eine -C(CN)-Gruppe in α-Stellung zum Substituenten A trägt.

3. Verfahren nach Anspruch 1 oder 2, dadurch gekennzeichnet, daß der Katalysator 0,1 bis 10 Gew.-% Palladium enthält.

4. Verfahren nach Anspruch 3, dadurch gekennzeichnet, daß der Katalysator 0,3 bis 5 Gew.-% Palladium enthält.

5. Verfahren nach einem der vorhergehenden Ansprüche, dadurch gekennzeichnet, daß man das sekundäre Amin III zum Nitril II im Molverhältnis von 1 : 1 bis 30 : 1 einsetzt.

6. Verfahren nach Anspruch 5, dadurch gekennzeichnet, daß man das sekundäre Amin III zum Nitril II im Molverhältnis von 1 : 1 bis 15 : 1 einsetzt.

7. Verfahren nach Anspruch 5, dadurch gekennzeichnet, daß man das sekundäre Amin III zum Nitril II im Molverhältnis von 1,1 : 1 bis 5 : 1 einsetzt.

8. Verfahren nach einem der vorhergehenden Ansprüche, dadurch gekennzeichnet, daß man die Umsetzung bei Temperaturen von 90 bis 200°C durchführt.

## Claims

1. A process for the preparation of peralkylated amines of the general formula I 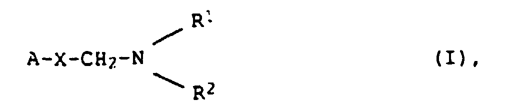 in which
R¹, R² are C₁- to C₂₀-alkyl, C₃- to C₈-cycloalkyl, C₄- to C₂₀-alkylcycloalkyl, C₄- to C₂₀-cycloalkylalkyl, C₂- to C₂₀-alkoxyalkyl, aryl, C₇- to C₂₀-alkylaryl, C₇- to C₂₀-aralkyl, C₂- to C₈-hydroxyalkyl, C₂- to C₈-mercaptoalkyl, C₈- to C₂₀-phenoxyalkyl, C₂- to C₈-aminoalkyl, C₂- to C₈-(NHR⁴)-alkyl, C₂- to C₈-(NR⁴R⁵)-alkyl or together are a saturated or unsaturated C₂- to C₆-alkylene chain which is optionally mono- to trisubstituted by C₁- to C₄-alkyl and optionally interrupted by oxygen or nitrogen,
X is a C₂- to C₂₀-alkylene or C₂- to C₂₀-alkenylene or C₄- to C₈-cycloalkylene, which is optionally mono- to pentasubstituted by R³, C₁- to C₈-alkyl, C₁- to C₈-alkoxy, C₁- to C₈-dialkylamino, phenoxy, diphenylamino and/or C₂- to C₈-alkoxycarbonyl,
A is hydrogen, C₁- to C₂₀-alkyl, C₃- to C₈-cycloalkyl, C₄- to C₂₀-alkylcycloalkyl, C₄- to C₂₀-cycloalkylalkyl, C₂- to C₂₀-alkoxyalkyl, aryl, C₇- to C₂₀-alkylaryl, C₇- to C₂₀-aralkyl, C₁- to C₂₀-alkoxy, hydroxyl, C₁- to C₂₀-hydroxyalkyl, amino, C₁- to C₂₀-alkylamino, C₂- to C₂₀-dialkylamino, C₃- to C₁₂-alkenylenamino, C₃- to C₈-cycloalkylamino, arylamino, aryl-C₁- to C₈-alkylamino, halogen, mercapto, C₂- to C₂₀-alkenylenoxy, C₃- to C₈-cycloalkoxy and aryloxy,
R³ is CH₂-NR¹R²,
R⁴, R⁵ are C₁- to C₂₀-alkyl,
where a nitrile of the general formula II
A-X-CN (II),
in which X and A have the abovementioned meanings, is added to a secondary amine of the general formula III 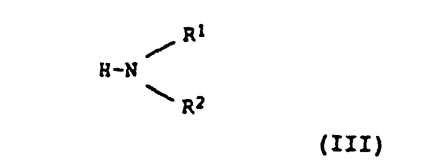 in which R¹ and R² have the abovementioned meanings, where R³ is -CH₂-NR¹R² or cyano,
and the mixture obtained is reacted with hydrogen at temperatures from 50 to 250°C and pressures from 5 to 350 bar in the presence of palladium as a catalyst, characterized in that the palladium is used on oxidic supports which are selected from γ-Al₂O₃, α-Al₂O₃, SiO₂, TiO₂ or ZrO₂ or are selected from γ-Al₂O₃, α-Al₂O₃, SiO₂, TiO₂ or ZrO₂ doped with alkali metal or alkaline earth metal.

2. A process as claimed in claim 1, characterized in that A is not hydrogen if X has a -C(CN) group in the α-position to the substituent A.

3. A process as claimed in claim 1 or 2, characterized in that the catalyst contains 0.1 to 10 % by weight of palladium.

4. A process as claimed in claim 3, characterized in that the catalyst contains 0.3 to 5% by weight of palladium.

5. A process as claimed in anyone of the preceding claims, characterized in that the secondary amine III is employed in a molar ratio from 1:1 to 30:1 relative to the nitrile II.

6. A process according to claim 5, characterized in that the secondary amine III is employed in a molar ratio from 1:1 to 15:1 relative to the nitrile II.

7. A process as claimed in claim 5, characterized in that the secondary amine III is employed in a molar ratio from 1.1:1 to 5:1 relative to the nitrile II.

8. A process as claimed in anyone of the preceding claims, characterized in that the reaction is carried out at temperatures from 90 to 200°C.

## Revendications

1. Procédé de préparation d'amines peralkylées de formule générale I 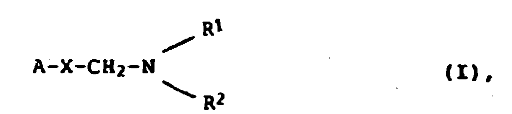 dans laquelle
R¹, R² représentent un groupement alkyle en C₁-C₂₀₀, cycloalkyle en C₃-C₈, alkyle-cycloalkyle en C₄-C₂₀, cycloalkyle-alkyle en C₄-C₂₀, alcoxy-alkyle en C₂-C₂₀, aryle, alkyle-aryle en C₇-C₂₀, aralkyle en C₇-C₂₀, hydroxyalkyle en C₂-C₈, mercaptoalkyle en C₂-C₈, phénoxyalkyle en C₈-C₂₀, aminoalkyle en C₂-C₈, (NHR⁴)-alkyle en C₂-C₈, (NR⁴R⁵)-alkyle en C₂-C₈, ou bien forment ensemble une chaine alkylène en C₂-C₆ saturée ou insaturée, éventuellement substituée une à trois fois par un groupement alkyle en C₁-C_{4,} éventuellement interrompue par de l'oxygène ou de l'azote,
x représente un groupement cycloalkylène en C₄-C₈ ou alcénylène en C₂-C₂₀ ou alkylène en C₂-C₂₀ éventuellement substitué une à cinq fois par R³, par un groupement alkyle en C₁-C₈. alcoxy en C₁-C₈, dialkylamino en C₁-C₈, phénoxy, diphénylamino et/ou alcoxycarbonyle en C₂-C₈,
A représente un atome d'hydrogène, un groupement alkyle en C₁-C₂₀, cyclolalkyle en C₃-C₈, cycloalkyle-alkyle en C₄-C₂₀, alkyle-cycloalkyle en C₄-C₂₀, alkyle-alcoxy en C₂-C₂₀, aryle, aryle-alkyle en C₇-C₂₀, aralkyle en C₇-C₂₀, alkoxy C₁-C₂₀, hydroxyle, hydroxyalkyle en C₁-C₂₀, amino, alkylamino en C₁-C₂₀, dialkylamino en C₂-C₂₀, alcénylènamino en C₃-C₁₂, cycloalkylamino en C₃-C₈, arylamino, aryl(alkyle en C₁-C₈)amino, un atome d'halogène, un groupement mercapto, alcénylènoxy en C₂-C₂₀, cycloalcoxy en C₃-C₈ et aryloxy,
R³ représente CH₂-NR¹R²,
R⁴, R⁵ représentent un groupement alkyle en C₁-C₂₀,
dans lequel on ajoute un nitrile de formule générale II
A - X - CN (II),
où X et A prennent les significations susmentionnées, à une amine secondaire de formule générale III 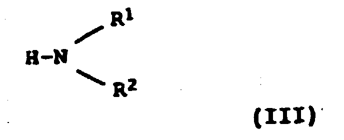 dans laquelle R¹ et R² prennent la signification susmentionnée, où R³ est mis pour -CH₂-NR¹R² ou un groupement cyano, puis l'on fait réagir le mélange obtenu avec de l'hydrogène, à des températures de 50-250°C et sous des pressions de 5-350 bar, en présence de palladium en tant que catalyseur, caractérisé en ce que l'on utilise du palladium sur des supports de type oxyde choisis parmi γ-Al₂O₃, α-Al₂O₃, SiO₂, TiO₂ ou ZrO₂ ou bien parmi γ-Al₂O₃, α-Al₂O₃, SiO₂, TiO₂ ou ZrO₂ dopés avec des alcalins ou des alcalino-terreux.

2. Procédé selon la revendication 1, caractérisé en ce que A n'est pas un atome d'hydrogène lorsque X porte un groupement -C(CN) en position α par rapport au substituant A.

3. Procédé selon la revendication 1 ou 2, caractérisé en ce que le catalyseur contient 0,1-10% en poids de palladium.

4. Procédé selon la revendication 3, caractérisé en ce que le catalyseur contient 0,3-5% en poids de palladium.

5. Procédé selon l'une quelconque des revendications précédentes, caractérisé en ce que l'on utilise l'amine secondaire III dans un rapport en moles de 1 : 1 à 30 : 1 par rapport au nitrile II.

6. Procédé selon la revendication 5, caractérisé en ce que l'on utilise l'amine secondaire III dans un rapport en moles de 1 : 1 à 15 : 1 par rapport au nitrile II.

7. Procédé selon la revendication 5, caractérisé en ce que l'on utilise l'amine secondaire III dans un rapport en moles de 1,1 : 1 à 5 : 1 par rapport au nitrile II.

8. Procédé selon l'une quelconque des revendications précédentes, caractérisé en ce que l'on mène la réaction à des températures de 90-200°C.
